(19) Europäisches Patentamt
European Patent Office
Office européen des brevets

(11) **EP 4 497 401 A1**

(12) **EUROPEAN PATENT APPLICATION**
published in accordance with Art. 153(4) EPC

(43) Date of publication:
**29.01.2025 Bulletin 2025/05**

(51) International Patent Classification (IPC):
***A61B 17/3207*** (2006.01)

(21) Application number: 23814976.9

(22) Date of filing: **18.05.2023**

(86) International application number:
**PCT/CN2023/094969**

(87) International publication number:
**WO 2023/231791 (07.12.2023 Gazette 2023/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.05.2022 CN 202210615844**

(71) Applicant: **Shanghai Microport Rotapace Medtech
Co., Ltd.
Shanghai 201203 (CN)**

(72) Inventors:
• **JI, Xiaofei**
**Shanghai 201203 (CN)**
• **CHANG, Zhaohua**
**Shanghai 201203 (CN)**
• **YUE, Bin**
**Shanghai 201203 (CN)**
• **YAO, Yingzhong**
**Shanghai 201203 (CN)**

(74) Representative: **Marks & Clerk LLP
15 Fetter Lane
London EC4A 1BW (GB)**

(54) **ROTATIONAL ATHERECTOMY APPARATUS AND ROTATIONAL ATHERECTOMY DEVICE**

(57) The present invention relates to a rotational atherectomy apparatus and a rotational atherectomy device. The rotational atherectomy apparatus includes a drive device and the rotational atherectomy device. The drive device is coupled to a drive shaft of the rotational atherectomy device in order to actuate rotation of the rotational atherectomy device. The rotational atherectomy device is used for vascular surgery and includes the drive shaft and a rotating abrasive head. The drive shaft has a distal connecting portion coupled to the rotating abrasive head. The rotating abrasive head and the drive shaft separately define hollow cavities axially extending therethrough. The hollow cavities are configured for insertion of a guide body therethrough. A centroid of a structure consisting of the rotating abrasive head and the connecting portion coupled thereto does not coincide with center axes of the hollow cavities. The present invention can reduce the risk of a surgical procedure using the rotational atherectomy apparatus to recanalize a blood vessel occluded by a lesion. It can also make the surgical procedure easier to perform.

Fig. 1

EP 4 497 401 A1

## Description

## TECHNICAL FIELD

The present invention relates to the field of medical devices, and particularly to a rotational atherectomy device and a rotational atherectomy apparatus.

## BACKGROUND

Atherosclerotic plaques are generally present in the coronary or peripheral vasculature and may have different characteristics depending on the texture. Heavily calcified lesions may require atherectomy pretreatment using a rotational atherectomy device, which can remove a calcified or fibrotic atherosclerotic plaque within a blood vessel by high-speed rotation and abrasion, thereby recanalizing the occluded blood vessel. The enlarged and smoothed vascular lumen allows easier subsequent implantation of a stent.

Existing rotational atherectomy devices mainly include a flexible drive shaft and a rotating abrasive head supported on the flexible drive shaft. The drive shaft drives high-speed rotation of the rotating abrasive head while pushing it forward into contact with and through a plaque, thereby abrading it away. The rotating abrasive head has a diameter not less than a diameter of the drive shaft. The rotating abrasive head is mostly disposed over a middle section of the drive shaft. In this case, there is a non-abrasive section of the drive shaft distally ahead of the rotating abrasive head, and this distal drive shaft section must be screwed or pushed through a stenotic lesion before the middle rotating abrasive head can be brought into contact with the lesion. This leads to poor atherectomy performance and crossability. Moreover, the distal drive shaft section may get stuck in the stenotic lesion during its screwing or pushing therethrough. This is risky and may even cause damage to the blood vessel due to excessive dilation. In particular, some lesions may be very stenotic or even completely occluded, and in these cases, it may be even difficult to bring the middle rotating abrasive head into contact with a lesion for abrasive atherectomy. Additionally, the rotating abrasive head may not be coated with an abrasive material over its entire surface and may therefore not be capable of atherectomy in both axial directions. Consequently, it is a frequent case that the rotating abrasive head can only abrade a lesion while being pushed forward therethrough, but cannot do the same thing while being retracted back through the lesion. This atherectomy approach is inefficient and associated with a high risk of the rotating abrasive head getting stuck in a vascular plaque and not being able to be moved anymore.

Apart from the above problems, the conventional rotating abrasive head cannot be adjusted in diameter during atherectomy of a lesion. Therefore, such atherectomy procedures typically involve the sequential use of multiple rotating abrasive heads in an order from the smallest diameter to the largest diameter. This, however, necessitates frequent interchanging of associated rotational atherectomy devices of different sizes, increasing the surgical time and the probability of blood vessel damage. In addition, a large rotating abrasive head diameter may add challenges to passage through a narrow blood vessel or catheter and arrival at a target vascular lesion in a surgical procedure, and may block blood flow in a stenotic blood vessel, as well as distal flow of a liquid coolant and/or lubricant. Some conventional rotational atherectomy devices include multiple rotating abrasive heads sequentially disposed over a middle drive shaft section from distal to proximal in an increasing diameter order. Although this can circumvent frequent interchanging, it requires the drive shaft to have an increased axial length. As a consequence, normal blood vessel segments on distal and proximal sides of the target lesion tend to be affected during atherectomy. Moreover, the problems of blocked blood flow and blocked flow of a liquid coolant and/or lubricant remain unsolved due to possible excessively large diameters of one or more proximal ones of the rotating abrasive heads. In some of these conventional devices, the multiple rotating abrasive heads are disposed eccentrically. However, due to differences among these rotating abrasive heads of various static diameters in terms of centroid, weight and/or the like, their angular momenta may vary during atherectomy. Consequently, the resulting centrifugal forces may interact in an uncontrolled manner, leading to uncontrolled movement of the rotating abrasive heads and hence their uncontrolled influence on a blood vessel. This adds uncertainties to the surgical procedure and reduces its safety. Furthermore, the use of multiple rotating abrasive heads requires greater overall drive shaft stiffness, which means reduced drive shaft compliance and hence more difficulties in passage through a narrow blood vessel or catheter and arrival at a target lesion.

## SUMMARY

It is an objective of the present invention to provide a rotational atherectomy device and a rotational atherectomy apparatus, which overcome at least one of the above-described problems associated with the conventional rotational atherectomy devices.

To this end, the present invention provides a rotational atherectomy device including a drive shaft and a rotating abrasive head, the drive shaft having a distal connecting portion connected to the rotating abrasive head, the rotating abrasive head and the drive shaft separately defining hollow cavities axially extending therethrough, the hollow cavities configured for insertion of a guide body therethrough, wherein a centroid of a structure consisting of the rotating abrasive head and the connecting portion connected thereto does not coincide with center axes of the hollow cavities.

In one embodiment, the rotating abrasive head has a distal portion, which has a diameter gradually increasing along its axis from distal to proximal and is coated on its outer surface with an abrasive layer.

In one embodiment, the rotating abrasive head has a proximal portion, which has a diameter gradually decreasing along its axis from distal to proximal and is coated on its outer surface with the abrasive layer. Additionally or alternatively, an intermediate portion of the rotating abrasive head between the distal and proximal portions is coated on its outer surface with the abrasive layer.

In one embodiment, the intermediate portion has a constant diameter or a diameter gradually increasing and then gradually decreasing along its axis from distal to proximal.

In one embodiment, the rotating abrasive head includes a base, which defines the hollow cavity of the rotating abrasive head and is coated on its outer surface with the abrasive layer. The abrasive layer is made of abrasive grains made of one or more abrasive materials.

In one embodiment, the abrasive layer has a thickness of 20-120 $\mu$m, such as 20 $\mu$m, 40 $\mu$m, 50 $\mu$m or 100 $\mu$m.

In one embodiment, the rotating abrasive head has a diameter gradually decreasing along its axis both distally and proximally. Accordingly, the rotating abrasive head may, for example, be shaped like a spindle wider in the middle and tapering toward the ends. Moreover, the rotating abrasive head may have a smooth outer surface.

In one embodiment, the rotating abrasive head includes at least one of:

a minimum diameter of the distal portion of the rotating abrasive head smaller than a minimum diameter of the proximal portion of the rotating abrasive head;
a maximum diameter of the distal portion of the rotating abrasive head smaller than a maximum diameter of the proximal portion of the rotating abrasive head; and
a radius of curvature of the distal portion of the rotating abrasive head greater than or equal to a radius of curvature of the proximal portion of the rotating abrasive head.

In one embodiment, the rotating abrasive head includes at least one of:

the distal portion of the rotating abrasive head having a diameter of 0.13-0.66 mm and for example, a minimum diameter of 0.13 mm, 0.2 mm or 0.3 mm and a maximum diameter of 0.66 mm;
a maximum diameter of 0.66-4.0 mm, such as 0.66 mm, 1.25 mm, 2.0 mm or 4.0 mm, of the intermediate portion between the proximal and distal portions of the rotating abrasive head;
the proximal portion of the rotating abrasive head having a diameter of 0.5-1.2 mm and for example, a minimum diameter of 0.5 mm, 0.75 mm or 1.0 mm and a maximum diameter of 1.2 mm.

In one embodiment, the rotational atherectomy device further includes the guide body, wherein the rotating abrasive head and the drive shaft are rotatable and axially movable relative to the guide body.

In one embodiment, the hollow cavity of the rotating abrasive head consists of distal and proximal hollow cavity sections in communication with each other, the distal hollow cavity section having a diameter matching a diameter of the guide body, the proximal hollow cavity section having a diameter greater than the diameter of the distal hollow cavity section, the proximal hollow cavity section secured to the connecting portion.

In one embodiment, the drive shaft is generally a hollow tubular structure with a constant diameter and has a center axis coincident with a center axis of the guide body.

In one embodiment, the drive shaft is a hollow tubular structure with a varying diameter, wherein the connecting portion has a diameter increasing along its axis from proximal to distal, and wherein the drive shaft further has a constant-diameter section joined to a proximal end of the connecting portion, the constant-diameter section having a center axis coincident with a center axis of the guide body.

In one embodiment, the connecting portion is inserted into the proximal hollow cavity section through a proximal opening of the rotating abrasive head, the proximal opening having a radial dimension smaller than or equal to a diameter of the connecting portion at its proximal end.

In one embodiment, the connecting portion is configured to be asymmetric about the center axis of the constant-diameter section. Addtionally or alternatively, the connecting portion has a diameter gradually increasing and then gradually decreasing along its axis from proximal to distal.

In one embodiment, the rotating abrasive head is spindle-shaped, wherein the proximal hollow cavity section has a matching spindle-like shape, and the connecting portion has a similar spindle-like shape complementary to that of the proximal hollow cavity section. In this embodiment, the rotating abrasive head may be configured as a hollow shell. This allows the rotating abrasive head to have a reduced weight, which can mitigate the influence of a centrifugal force on movement of the rotating abrasive head.

To the above end, the present invention also provides a rotational atherectomy apparatus including a drive device and the rotational atherectomy device as defined above. The drive device is coupled to the drive shaft in the rotational atherectomy device and configured to actuate rotation of the rotational atherectomy device.

The present invention provides a rotational atherectomy device and a rotational atherectomy apparatus. The rotational atherectomy device includes a drive shaft and a rotating abrasive head. The drive shaft has a distal connecting portion coupled to the rotating abrasive head. The rotating abrasive head and the drive shaft separately define hollow cavities axially extending therethrough. The hollow cavities are configured for insertion of a guide body therethrough. A centroid of a structure consisting of the rotating abrasive head and the connecting portion coupled thereto does not coincide with center axes of the hollow cavities. With this arrangement, the present invention has at least the following advantages: First, arranging the rotating abrasive head at the distal end of the drive shaft imparts atherectomy capabilities to the distal end of the rotational atherectomy device. Accordingly, in a surgical procedure, it is made unnecessary to screw or push a distal drive shaft section through a stenotic lesion. Instead, the rotating abrasive head can be directly brought into contact with the lesion for atherectomy. In this way, better atherectomy performance and crossability can be achieved, reducing the risk of the surgical procedure and increasing its success rate.

Second, deviating the centroid of the structure consisting of the connecting portion of the drive shaft and the rotating abrasive head coupled thereto enables the rotating abrasive head to adapt a diameter of atherectomy to a lesion in need of atherectomy and recanalization, thereby allowing for effective removal of the lesion. This dispenses with the use of multiple rotating abrasive heads of various sizes, which requires frequent interchanging of associated devices and a longer surgical time and is associated with a higher surgical risk. In particular, the rotating abrasive head is allowed to have a smaller static diameter (i.e., a diameter in a non-rotating state), which enables the rotational atherectomy device to be more easily advanced through a narrow blood vessel and catheter to a target vascular lesion, reducing surgical difficulties and improving surgical treatment.

Third, a smaller static diameter of the rotating abrasive head is associated with a lower probability of the rotating abrasive head blocking blood flow in a stenotic blood vessel or blocking distal flow of a liquid coolant and/or lubricant. This further reduces the risk associated with a surgical procedure and increases its safety.

Fourth, as only one rotating abrasive head is included and disposed at the distal end of the drive shaft, the drive shaft is allowed to have a reduced axial length, which mitigates the influence of the drive shaft on distal side of a lesion in need of atherectomy and normal blood vessel segments on proximal side. In particular, the single rotating abrasive head is eccentrically disposed to allow for controlled centrifugation during atherectomy, which does not adversely affect movement of the rotating abrasive head or a blood vessel. In this way, uncertainties associated with a surgical procedure using the device can be reduced, increasing safety of the surgical procedure. Moreover, the use of only one rotating abrasive head does not increase overall stiffness of the drive shaft and ensures that it is compliant enough to facilitate its advancement through a blood vessel and catheter.

Apart from the above benefits, in the rotational atherectomy device and apparatus of the present invention, the distal portion of the rotating abrasive head is preferred to have a diameter gradually increasing along its axis from distal to proximal, and the outer surface of the distal portion of the rotating abrasive head is coated with an abrasive layer. This abrasive layer on the distal portion of the rotating abrasive head may be directly brought into contact with a lesion in need of atherectomy and can thereby abrade the lesion more efficiently. In addition, the proximal portion of the rotating abrasive head may have a diameter gradually decreasing along its axis from distal to proximal, and the outer surface of the proximal portion of the rotating abrasive head is coated with the abrasive layer. In this way, the rotating abrasive head is capable of bidirectional atherectomy. Thus, even when the rotating abrasive head gets stuck in a lesion after it drills through the lesion, it can be retracted while abrading the lesion. As a result, not only more efficient atherectomy can be achieved, the risk of a surgical procedure using the device can be further reduced.

**BRIEF DESCRIPTION OF THE DRAWINGS**

Those of ordinary skill in the art would appreciate that the following drawings are presented to enable a better understanding of the present invention and do not limit the scope thereof in any sense, in which:

Fig. 1 is a perspective view of a rotational atherectomy device according to an embodiment of the present invention;
Fig. 2 is a cross-sectional view of a rotational atherectomy device according to an embodiment of the present invention;
Fig. 3 is an end view showing a centroid of a structure consisting of a connecting portion and a rotating abrasive head coupled thereto being deviated from center axes of hollow cavities according to an embodiment of the present invention;
Fig. 4 illustrates how a rotating abrasive head in a rotational atherectomy device according to an embodiment of the present invention comes into contact with a lesion first for atherectomy and recanalization during use;
Fig. 5 shows a rotating abrasive head having been advanced on a guide body through a stenotic lesion during use of a rotational atherectomy device according to an embodiment of the present invention;
Fig. 6 shows a drive shaft having a distal connecting portion, which has an increased diameter and is fixedly coupled to a hollow rotating abrasive head, in a rotational atherectomy device according to an embodiment of the present invention;
Fig. 7 shows variation of a centrifugal force on a rotating abrasive head as a function of a speed of rotation according to an embodiment of the present invention; and
Fig. 8 shows variation of a centrifugal force on a rotating abrasive head as a function of a diameter of atherectomy according to an embodiment of the present invention.

List of Reference Numerals

10 rotational atherectomy device; 11 rotating abrasive head; 11a distal portion; 11b proximal portion; 11c intermediate portion; 111 abrasive layer; 112 base; 113 proximal hollow cavity section; 114 distal hollow cavity section; 12 drive shaft; 121 connecting portion; 122 constant-diameter section; 13 guide body; 14 centroid; 31 blood vessel; 32 stenotic lesion; 41 catheter.

## DETAILED DESCRIPTION

Objectives, advantages and features of the present invention will become more apparent upon reading the following more detailed description thereof in conjunction with the accompanying drawings. Note that the figures are provided in a very simplified form not necessarily drawn to exact scale for the only purpose of helping explain embodiments of the invention disclosed herein in a more convenient and clearer way.

As used herein, the singular forms "a", "an" and "the" include plural referents, unless the context clearly dictates otherwise. As used herein, the term "or" is generally employed in the sense including "and/or", unless the context clearly dictates otherwise. As used herein, the term "plurality" means "two or more", and the term "several" refers to a non-limited number, unless otherwise specified. The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the invention. In the following, for ease of description, the terms "distal", "proximal", "axial" and "circumferential" may be used. "Distal" refers to a side away from an operator operating a rotational atherectomy apparatus, while "proximal" refers to a side closer to the operator. "Axial" refers to a direction along a center axis of the rotational atherectomy apparatus, or of a rotational atherectomy device thereof, while "circumferential" refers to a direction about the axis. The center axis is oriented in a lengthwise direction of the rotational atherectomy device, or of a blood vessel in which the device is deployed. As used herein, the term "diameter" refers to an outer diameter of a structure. A diameter of a rotating abrasive head includes that of an abrasive layer thereof.

Preferred embodiments of the present invention will be described in greater detail below with reference to the accompanying drawings. If there is no conflict, the embodiments described below and features thereof can complement or be combined with each other.

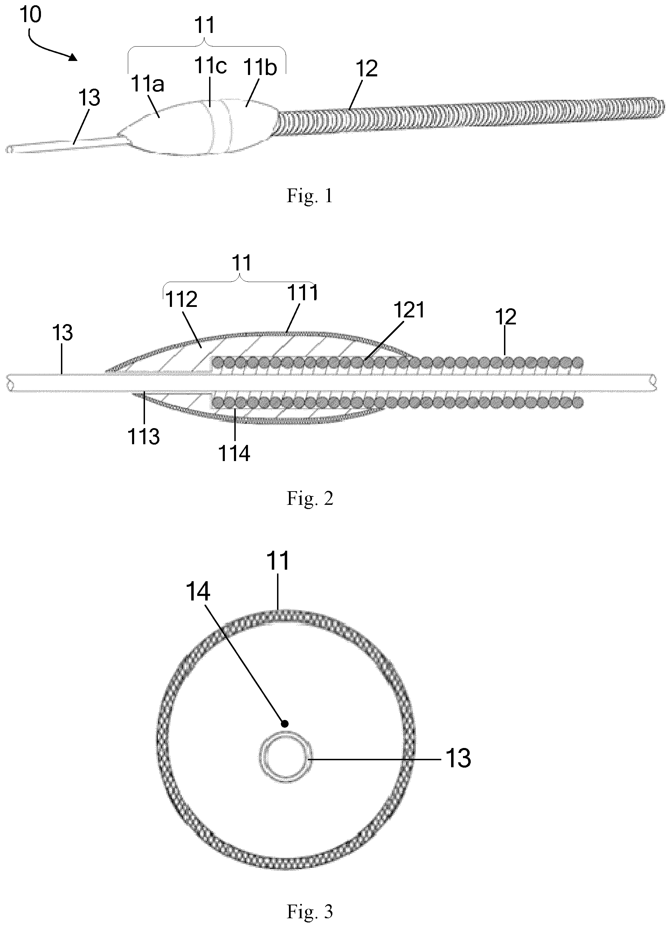

As shown in Figs. 1 to 8, in some embodiments of the present invention, there are disclosed a rotational atherectomy apparatus and a rotational atherectomy device thereof. The rotational atherectomy apparatus includes a rotational atherectomy device 10 and a drive device (not shown). The drive device is coupled to a proximal end of a drive shaft 12 in the rotational atherectomy device 10 and is thereby able to actuate rotation of the rotational atherectomy device 10. The rotational atherectomy apparatus and the rotational atherectomy device thereof are used for vascular surgery for, for example, removing tissue from a body channel. For example, the rotational atherectomy device may be used to remove a calcified or fibrotic atherosclerotic plaque from a blood vessel (e.g., a coronary, peripheral or other blood vessel), thereby recanalizing the blood vessel occluded by the plaque and smoothing its inner side.

The rotational atherectomy device 10 includes a rotating abrasive head 11 and the drive shaft 12. The drive shaft 12 has a distal connecting portion 121, and the rotating abrasive head 11 is coupled to the connecting portion 121. Generally, the rotating abrasive head 11 and the drive shaft 12 are separately fabricated and then assembled together. The drive shaft 12 is an integral one-piece structure. Preferably, the drive shaft 12 is a flexible drive shaft, which may be implemented as a spring coil. The drive shaft 12 may be actuated by the drive device to rotate, causing rotation of the rotating abrasive head 11.

Differing from the conventional devices, in which a rotating abrasive head is disposed over a middle portion of a drive shaft, according to the present invention, the rotating abrasive head 11 is disposed at the distal end of the drive shaft 12. In this way, the rotating abrasive head 11 can first come into contact with a vascular lesion and drill into a stenotic portion thereof, overcoming the problem associated with the conventional device that the middle rotating abrasive head can come into contact with a stenotic lesion only after a distal section of the drive shaft is screwed or pushed through the lesion. Therefore, the present invention can achieve better atherectomy performance and crossability can be achieved and reduce the risk associated with a surgical procedure. Moreover, as only one rotating abrasive head 11 may be included, an overall axial length of the drive shaft 12 is reduced, reducing the influence of the drive shaft 12 on distal side of a lesion in need of atherectomy and normal blood vessel segments on proximal side and thereby additionally reducing the risk associated with a surgical procedure.

In addition, both the rotating abrasive head 11 and the drive shaft 12 define hollow cavities axial extending therethrough, a guide body 13 can be inserted through the hollow cavities of the rotating abrasive head 11 and the drive shaft 12. The guide body 13 may be, for example, a guidewire for guiding the rotational atherectomy device 10 through a diseased blood vessel. Center axes of the hollow cavities of the rotating abrasive head 11 and the drive shaft 12 coincide with each other.

The rotating abrasive head 11 may be coupled to the connecting portion 121 so that a centroid 14 of the resulting coupled structure does not coincide with the center axes of the hollow cavities. In this case, as the rotating abrasive head 11 and the drive shaft 12 are spun about the center axes of the hollow cavities, the rotating abrasive head 11 will also revolve under the action of a centrifugal force about an axis deviated from the center axes of the hollow cavities. Moreover, speeding up the rotation of the rotating abrasive head 11 (i.e., increasing its speed of rotation n) can increase the diameter of revolution of the rotating abrasive head 11 (i.e., its diameter of atherectomy D). The diameter of atherectomy D is greater than a static diameter of the rotating abrasive head 11. With this arrangement, the rotating abrasive head 11 can adapt the diameter of atherectomy D to lesions of various sizes in need of atherectomy and recanalization. In this way, it can remove various lesions in a desired manner, while avoiding the use of multiple rotating abrasive heads of different sizes and hence frequent interchanging of them. This allows a surgical procedure using the device to be completed within a shorter time and associated with a lower risk. Moreover, the rotating abrasive head 11 is allowed to have an even smaller static diameter, which enables the rotational atherectomy device 10 to be more easily advanced through a narrow blood vessel and catheter to a target site in a diseased blood vessel, reducing surgical difficulties. Further, it will be understood that, a smaller static diameter of the rotating abrasive head 11 is associated with a lower probability of the rotating abrasive head 11 blocking blood flow in a stenotic blood vessel or blocking distal flow of a liquid coolant and/or lubricant. This further reduces the risk associated with a surgical procedure and increases its safety.

It will be understood that, since only one rotating abrasive head 11 may be used and the single rotating abrasive head 11 can be disposed at the distal end of the drive shaft 12, an overall axial length of the drive shaft 12 is reduced, and the influence of the drive shaft 12 on distal side of a lesion in need of atherectomy and normal blood vessel segments on proximal side can be induced during atherectomy. In particular, the single rotating abrasive head 11 may be eccentrically disposed to allow for controlled centrifugation during atherectomy, which does not adversely affect movement of the rotating abrasive head or a blood vessel. In this way, uncertainties associated with a surgical procedure using the device can be reduced, increasing safety of the surgical procedure. Moreover, the use of only one rotating abrasive head 11 does not increase overall stiffness of the drive shaft 12 and ensures that it is compliant enough to facilitate its advancement through a blood vessel and catheter. It will be understood that, as used herein, the term "static" refers to a non-rotating state of the rotating abrasive head 11. Accordingly, the term "static diameter" refers to a diameter of the rotating abrasive head 11 in the non-rotating state. Further, the diameter of atherectomy D is a diameter of revolution of the rotating abrasive head 11.

In practical use, the diameter of revolution (i.e., the diameter of atherectomy) of the rotating abrasive head 11 can be adjusted by changing the speed of rotation of the rotating abrasive head 11. Accordingly, the diameter of atherectomy D of the rotating abrasive head 11 can be varied, for example, increased or reduced, to adapt the single rotating abrasive head 11 to lesions of various sizes, for example, both big and small lesions.

In one embodiment, a centrifugal force on the rotating abrasive head 11 may be calculated according to:

$$F_C = m\Delta x(\pi n/30)^2$$

where Fc represents the centrifugal force; m is the weight of the structure consisting of the connecting portion 121 and the rotating abrasive head 11 coupled thereto; Δx is a distance that the centroid 14 of the structure consisting of the connecting portion 121 and the rotating abrasive head 11 coupled thereto is deviated from the center axes of the hollow cavities; and n is the speed of rotation of the rotating abrasive head 11. The present invention is not limited to any particular values of these parameters, and those skilled in the art would know how to calculate a centrifugal force according to the above formula and how it varies as a function of the various factors. Therefore, the centrifugal force $F_C$ on the rotating abrasive head 11 is related to the weight m of the structure, the deviation of its centroid and the speed of rotation of the rotating abrasive head.

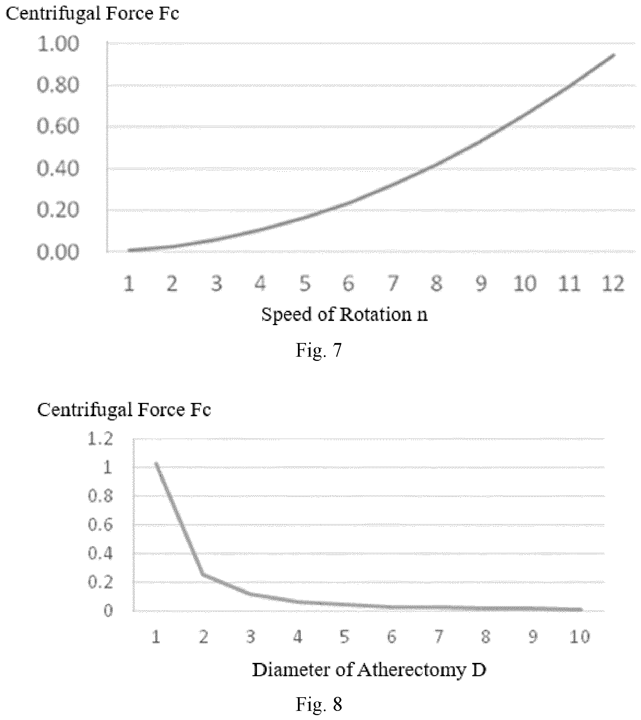

It will also be understood that the center axes of the hollow cavities correspond to an axis of spin, and a center axis of a diseased blood vessel in which the device is deployed corresponds to an axis of revolution. It will also be understood that as the speed of rotation n increases, the distance between the axis of revolution and the centroid 14 varies. Therefore, the diameter of atherectomy D increases with the speed of rotation n. However, during atherectomy of a stenotic lesion 32 using the rotating abrasive head 11 rotating a given speed n, the diameter of atherectomy D increases, but the centrifugal force Fc decreases. Moreover, when the diameter of atherectomy D increases and reaches a maximum diameter, the centrifugal force Fc tends to 0. Therefore, no damage will be caused to any normal blood vessel segment. Reference can be made to Figs. 7 and 8 for more details of this process.

As shown in Fig. 7, as the speed of rotation n increases, the centrifugal force $F_C$ on the rotating abrasive head 11 also increases. Accordingly, the centrifugal force Fc, and hence the diameter of atherectomy D, can be increased by adjusting the speed of rotation n, adapting the device to a larger lesion. As shown in Fig. 8, at a given speed of rotation n, as the diameter of atherectomy D of the rotating abrasive head increases, the centrifugal force Fc acting thereon decreases and tends to 0. Finally, the diameter of atherectomy D becomes increasingly constant and reaches a maximum diameter. At the same time, the centrifugal force Fc tends to 0 or becomes very weak, and the rotating abrasive head 11 will cause no damage to any normal blood vessel segment. Therefore, throughout the atherectomy process, the centrifugal force remains in a predictable, controlled range, making movement of the rotating abrasive head 11 and the force's influence on a blood vessel controllable and ensuring surgical safety.

In one embodiment, as shown in Figs. 1 to 3, the deviation of the centroid 14 from the center axes of the hollow cavities is accomplished by structural asymmetry of the rotating abrasive head 11 about the center axes of the hollow cavities. For example, as viewed from the perspective of Fig. 2, a portion of the rotating abrasive head 11 above the guide body 13 may have a greater weight than the rest thereof below the guide body 13, and the drive shaft 12 may be generally a constant-diameter hollow tubular structure with a center axis coincident with a center axis of the guide body 13. In this case, due to the structural asymmetry of the rotating abrasive head 11, the centroid 14 is located above the center axes of the hollow cavities for insertion of the guide body 13 therethrough.

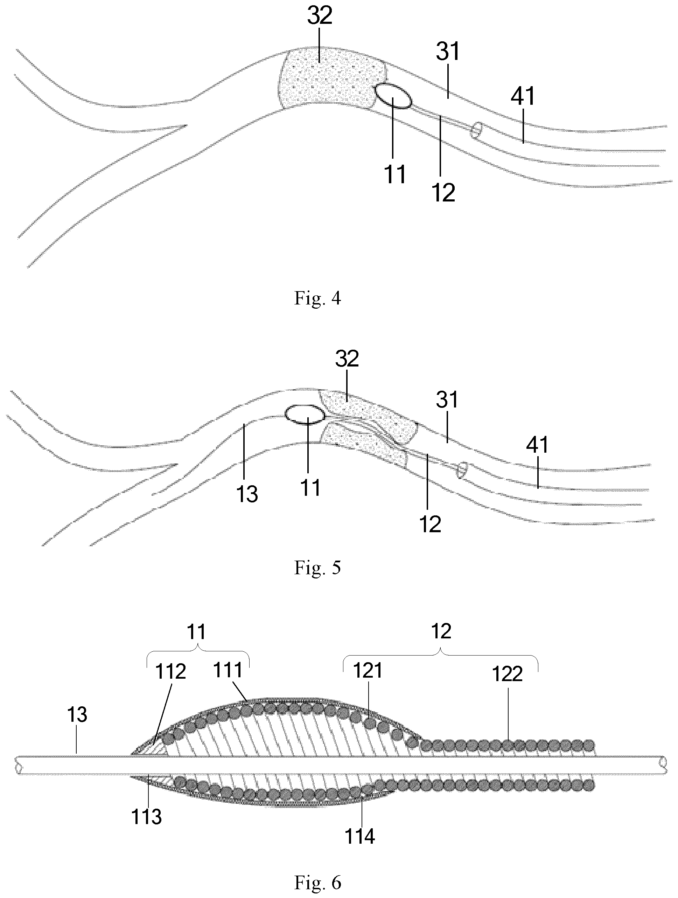

In an alternative embodiment, as shown in Fig. 6, the deviation of the centroid 14 from the center axes of the hollow cavities is accomplished by structural asymmetry of the rotating abrasive head 11 about the center axes of the hollow cavities. For example, as viewed from the perspective of Fig. 6, a portion of the rotating abrasive head 11 above the guide body 13 may have a greater weight than the rest thereof below the guide body 13, and the drive shaft 12 may be a varying-diameter hollow tubular structure. The connecting portion 121 may have a diameter, which axially increases from proximal to distal. The drive shaft 12 may further include a constant-diameter section 122 joined to a proximal end of the connecting portion 121. In this case, the center axis of the drive shaft 12 may partially coincide with that of the guide body 13. For example, the constant-diameter section 122 may have a center axis coincident with that of the guide body 13, while the connecting portion 121 may be asymmetric about the center axes of the hollow cavities. Due to both the structural asymmetry of the rotating abrasive head 11 and the structural asymmetry of the connecting portion 121, the centroid 14 of the structure consisting of the connecting portion 121 and the rotating abrasive head 11 coupled thereto is located above the center axes of the hollow cavities for insertion of the guide body 13 therethrough. However, in further alternative embodiments of this application, the connecting portion 121 may be symmetrically configured about the center axis of the constant-diameter section 122, i.e., about the center axes of the hollow cavities.

In order to enable more effective removal of a lesion, a distal portion 11a of the rotating abrasive head 11 may have a diameter gradually increasing along its axis from distal to proximal. Accordingly, the distal portion 11a of the rotating abrasive head 11 may have a smooth tapered surface, which can facilitate passage through a lesion. Moreover, the distal portion 11a of the rotating abrasive head 11 may be coated on its outer surface with an abrasive layer 111 (see Figs. 2 and 6). Once the distal portion 11a of the rotating abrasive head 11 comes into contact with a lesion, it can start abrasively removing the lesion with high efficiency using the abrasive layer 111.

Conventional rotating abrasive heads can only abrade a lesion while being pushed forward therethrough, but cannot do the same thing while being retracted back through the lesion. In view of this, a proximal portion 11b of the rotating abrasive head 11 has a diameter gradually decreasing along its axis from distal to proximal. Accordingly, the proximal portion 11b of the rotating abrasive head 11 may have a smooth tapered surface, which can facilitate passage through a lesion during retraction. Moreover, the proximal portion 11b of the rotating abrasive head 11 may also be coated on its outer surface with the abrasive layer 111 (see Figs. 2 and 6). With this arrangement, the rotating abrasive head 11 can provide bidirectional atherectomy in both distal, forward and proximal, backward directions. Thus, even when the rotating abrasive head 11 gets stuck in a lesion during its forward movement under the action of a pushing force, it can be retracted while abrading the lesion. As a result, not only more efficient atherectomy can be achieved, but the rotating abrasive head 11 can be prevented from getting stuck in a vascular plaque and being not able to move anymore, further reducing the risk of a surgical procedure.

Preferably, the entire outer surface of the rotating abrasive head 11 is coated with the abrasive layer 111. In one embodiment, in addition to the proximal portion 11b and the distal portion 11a, an intermediate portion 11c of the rotating abrasive head 11 between the distal portion 11a and the proximal portion 11b is also coated on its outer surface with the abrasive layer 111. This allows for even more efficient atherectomy. The intermediate portion 11c may have a constant diameter, hence the intermediate portion 11c has a cylindrical shape. That is, the rotating abrasive head 11 may be cylindrical in the middle and tapered at both ends. Accordingly, the intermediate portion 11c may have a diameter, which is equal to maximum diameters of the proximal portion 11b and the distal portion 11a. Alternatively, the intermediate portion 11c may have a diameter, which gradually increases and then gradually decreases along its axis from distal to proximal. In this case, the outer surface of the intermediate portion 11c may be curved, and the rotating abrasive head 11 may be generally spindle-shaped. Moreover, a diameter of the intermediate portion 11c at its distal end may be equal to a maximum diameter of the distal portion 11a at its proximal end, and a diameter of the intermediate portion 11c at its proximal end may be equal to a maximum diameter of the proximal portion 11b at its distal end, as shown in Figs. 1 to 6.

In a specific embodiment, the rotating abrasive head 11 may include a base 112, the base 112 may define the aforementioned hollow cavity of the rotating abrasive head 11. The abrasive layer 111 may be coated on an outer surface of the base 112. The abrasive layer 111 may be composed of abrasive grains, which may be made of one or more abrasive materials. The abrasive grains may be fixed to the base 112 by being partially embedded therein. A thickness of the abrasive layer 111 may be in the range of 20-120 μm, such as 20 μm, 40 μm, 50 μm or 100 μm. It will be understood that the thickness of the abrasive layer 111 refers to its thickness above a surface of the base 112. Desirably, the thickness of the abrasive layer 111 is not too large or too small. The larger the thickness of the abrasive layer 111, the larger the static diameter of the rotating abrasive head. An excessively thickness of the abrasive layer 111 is not conducive to atherectomy performance.

The present invention is not limited to any particular material of the abrasive grains. The abrasive grains may be made of one or more suitable abrasive materials. As an illustrative example, the abrasive grains may be made of one or more of diamond, fused silica, titanium nitride, tungsten carbide, silicon carbide, etc. The present invention is not limited to any particular material of the base 112. The base 112 may be made of one or more suitable materials, such as metallic and non-metallic materials. As an illustrative example, the base 112 may be made of one or more metallic materials such as stainless steel, nickel, etc. The material of the base 112 may further include a radiopaque material, for example, one or more of radiopaque materials such as tungsten, platinum, iridium, etc.

In order to make the rotating abrasive head 11 more powerful in passing through a stenotic lesion, the rotating abrasive head 11 is preferred to have a diameter gradually decreasing along its axis both distally and proximally. The gradually distally decreasing diameter of the rotating abrasive head 11 along its axis can facilitate atherectomy by the distal portion 11a in the forward direction. Moreover, since the diameter of the distal portion 11a is smaller than that of the intermediate portion 11c, the distal portion 11a of the rotating abrasive head 11 can more easily first come into contact with a lesion in need of atherectomy. The gradually proximally decreasing diameter of the rotating abrasive head 11 along its axis enables atherectomy by the proximal portion 11b during its retraction in the backward direction. Moreover, since the diameter of the proximal portion 11b is smaller than that of the intermediate portion 11c, the proximal portion 11b of the rotating abrasive head 11 can more easily first come into contact with a lesion in need of atherectomy during its retraction. Further, a minimum diameter of the distal portion 11a of the rotating abrasive head 11 may be smaller than a minimum diameter of the proximal portion 11b of the rotating abrasive head 11. With this arrangement, the rotating abrasive head 11 is narrowest at its distal end. This can additionally facilitate advancement of the rotating abrasive head 11 through a narrow blood vessel and catheter and its arrival at a target vascular lesion. Furthermore, the maximum diameter of the distal portion 11a of the rotating abrasive head 11 may be smaller than that of the proximal portion 11b of the rotating abrasive head 11. This allows the distal portion 11a to have a greater lengthwise extent, which is also conducive to advancement of the rotating abrasive head 11 through a narrow blood vessel and catheter and its arrival at a target vascular lesion. For example, in the illustrated embodiment, the rotating abrasive head 11 may be shaped like a spindle wider in the middle and tapering toward the ends. The spindle-shaped rotating abrasive head 11 may have a smooth outer surface and define certain degrees of curvature at the ends.

In general, the diameter of the distal portion 11a of the rotating abrasive head 11 may be configured based on a diameter of the guide body 13. In one embodiment, the diameter of the distal portion 11a of the rotating abrasive head 11 may be 0.13-0.66 mm. For example, the minimum diameter of the distal portion 11a may be configured to be 0.13 mm, 0.2 mm or 0.3 mm, and the maximum diameter thereof may be configured to be 0.66 mm.

In general, the diameter of the proximal portion 11b of the rotating abrasive head 11 may also be configured based on the diameter of the drive shaft 12. In one embodiment, the diameter of the proximal portion 11b of the rotating abrasive head 11 may be 0.5-1.2 mm. For example, the minimum diameter of the proximal portion 11b may be configured to be 0.5 mm, 0.7 mm or 1.0 mm, and the maximum diameter thereof may be configured to be 1.2 mm.

Considering that the rotating abrasive head 11 accommodates both the guide body 13 and the connecting portion 121 of the drive shaft 12 that it is coupled to, the guide body 13 is inserted through the drive shaft 12, the intermediate portion 11c of the rotating abrasive head 11 may have a maximum diameter configured based on the diameters of both the guide body 13 and the drive shaft 12. In one embodiment, the maximum diameter of the intermediate portion 11c of the rotating abrasive head 11 may be 0.66-4.0 mm, such as 0.66 mm, 1.25 mm, 2.0mm or 4.0 mm. It will also be understood that the intermediate portion 11c of the rotating abrasive head 11 should not be narrowly interpreted as being defined absolutely at the middle thereof; rather, it may be defined at any location between the distal portion 11a and the proximal portion 11b.

In a preferred embodiment, the guide body 13 may form part of the rotational atherectomy device 10, the guide body 13 be configured to be inserted through the hollow cavities of both the drive shaft 12 and the rotating abrasive head 11, the guide body 13 protrude out of the distal end of the rotating abrasive head 11. The rotating abrasive head 11 and the drive shaft 12 may be rotatable and axially movable relative to the guide body 13 to allow the drive shaft 12 to translate and rotate on the guide body 13. However, in alternative embodiments of the present application, the guide body 13 may be provided as a separate component for use in cooperation with the rotational atherectomy device 10.

In order to ensure sufficiently strong coupling of the rotating abrasive head 11 to the distal end of the drive shaft 12, the coupling is generally accomplished by inserting the connecting portion 121 of the drive shaft 12 into the hollow cavity of the rotating abrasive head 11. In one embodiment, the hollow cavity of the rotating abrasive head 11 may consist of distal and proximal hollow cavity sections in communication with each other. The distal hollow cavity section may have a diameter matching the diameter of the guide body 13, and the proximal hollow cavity section may have a diameter greater than the diameter of the distal hollow cavity section. Moreover, the connecting portion 121 may be secured to the proximal hollow cavity section. It will be understood that the distal hollow cavity section is configured for insertion of the guide body 13 therethrough, and the proximal hollow cavity section is configured for insertion of the connecting portion 121 of the drive shaft 12 therethrough, with the guide body 13 being in turn inserted through the drive shaft 12.

Figs. 1 and 2 show an optional embodiment of coupling the drive shaft 12 to the rotating abrasive head 11. In this exemplary embodiment, the hollow cavity of the rotating abrasive head 11 consists of a distal hollow cavity section 113 and a proximal hollow cavity section 114, which communicate with each other. Both the distal cavity section 113 and the proximal cavity section 114 are cylindrical and their center axes coincide with each other. The distal hollow cavity section 113 has a diameter matching a diameter of a guidewire, and the proximal hollow cavity section 114 has a diameter matching an overall diameter of the drive shaft 12. Additionally, the drive shaft 12 is a constant-diameter hollow tubular structure, and the connecting portion 121 of the drive shaft 12 is inserted into the proximal hollow cavity section 114 and secured to the proximal hollow cavity section 114, for example, the connecting portion 121 is secured to the proximal hollow cavity section 114 by crimping. Here, by "crimping", it is intended to mean that the diameter of the drive shaft 12 may be greater than that of the proximal hollow cavity section 114 and the connecting portion 121 may be secured to the proximal hollow cavity section 114 by being deformed and inserted therein. In this embodiment, center axes of the distal cavity section 113 and the proximal cavity section 114 both coincide with the center axis of the drive shaft 12.

However, the present invention is not limited to any method of coupling the rotating abrasive head 11 to the connecting portion 121. Apart from crimping, the coupling may also be accomplished by, for example, welding, gluing or other mechanical coupling methods.

In an alternative embodiment, the drive shaft 12 may be a varying-diameter hollow tubular structure. Fig. 6 shows another optional embodiment of coupling the drive shaft 12 to the rotating abrasive head 11. In this exemplary embodiment, the connecting portion 121 is inserted into the proximal hollow cavity section 114 through a proximal opening of the rotating abrasive head 11. The proximal opening has a radial dimension smaller than or equal to the diameter of the proximal end of the connecting portion 121, thereby restricting the drive shaft 12 from moving away from the rotating abrasive head 11. In this way, the drive shaft 12 is prevented from separation from the rotating abrasive head 11 during an atherectomy procedure. Additionally, the connecting portion 121 may be welded, glued or otherwise secured to the proximal hollow cavity section 114. In this embodiment, the center axes of the distal cavity section 113 and the proximal cavity section 114 both coincide with the center axis of the drive shaft 12.

Further, when the rotating abrasive head 11 is spindle-shaped, the proximal hollow cavity section 114 may have a matching spindle-like shape, and the connecting portion 121 may have a similar spindle-like shape complementary to that of the proximal hollow cavity section 114. For example, the diameter of the connecting portion 121 may gradually increase and then gradually decrease along its axis from proximal to distal. In this case, the rotating abrasive head 11 may be configured as a hollow shell. This allows the rotating abrasive head 11 to have a reduced weight, which can mitigate the influence of a centrifugal force on movement of the rotating abrasive head 11.

The distal portion 11a and proximal portion 11b of the rotating abrasive head 11 may have the same or different radii of curvature. In general, the radius of curvature of the distal portion 11a of the rotating abrasive head 11 may be greater than or equal to that of the proximal portion 11b of the rotating abrasive head 11. The radius of curvature of the distal portion 11a of the rotating abrasive head 11 is preferred to be greater than that of the proximal portion 11b of the rotating abrasive head 11 because this allows the distal portion 11a to be less steep and can more easily pass through a lesion.

The structure of the rotating abrasive head 11 is further explained below with respect to specific embodiments.

Figs. 1 to 3 show an exemplary embodiment of the rotating abrasive head 11. As shown in Figs. 1 to 3, the abrasive layer 111 has an average thickness of 50 μm, and the abrasive layer 111 is made of diamond abrasive grains. The base 112 is made of a combination of stainless steel and nickel, and the rotating abrasive head 11 has a spindle-like shape tapered at both ends. Moreover, the distal portion 11a of the rotating abrasive head 11 defines a minimum diameter, which allows for better atherectomy and drilling into a stenotic portion. The radius of curvature of the distal portion 11a of the rotating abrasive head 11 is 4.5 mm, and the radius of curvature of the proximal portion 11b of the rotating abrasive head 11 is 3.5 mm. The minimum diameter of the distal portion 11a of the rotating abrasive head 11 is 0.30 mm, and the maximum diameter of the intermediate portion 11c of the rotating abrasive head 11 is 1.25 mm. The minimum diameter of the proximal portion 11b of the rotating abrasive head 11 is 0.75 mm. In practical use, after the rotating abrasive head 11 drills through a lesion, it may be further caused to revolve under the action of a centrifugal force to increase the diameter of atherectomy. Since there is only a single rotating abrasive head 11 provided at the distal end of the drive shaft 12, the distance the centroid 14 is deviated from the center axes of the hollow cavities will not change due to absence of interference from any other rotating abrasive head 11. Therefore, both the centrifugal force and the diameter of revolution can be stably controlled, making the atherectomy process controllable.

In more detail, as shown in Fig. 4, the rotating abrasive head 11 coupled to the connecting portion 121 of the drive shaft 12 may be deployed in a blood vessel 31, the entire outer surface of the rotating abrasive head 11 is coated with the abrasive layer 111, the distal portion 11a of the rotating abrasive head 11 has a minimum diameter. The abrasive layer 111 on the distal portion 11a of the rotating abrasive head 11 may first come into contact with a stenotic lesion 32 for atherectomy and recanalization. As shown in Fig. 5, in a conventional scenario, due to elasticity of the blood vessel 31, a rotational atherectomy device 10 may be advanced on the guide body 13 through the stenotic lesion 32 as a result of being pushed distally by an operator. At this time, since a diameter defined by the stenotic lesion 32 may be smaller than a maximum diameter of the rotating abrasive head 11, the rotating abrasive head 11 may get stuck in the stenotic lesion 32 and not be able to be retracted back through the lesion. In contrast, according to the present invention, as the proximal portion of the rotating abrasive head 11 is also tapered and covered with the abrasive layer 111, it is capable of atherectomy and recanalization while being retracted backward.

Fig. 6 shows another exemplary embodiment of the rotating abrasive head 11. As shown in Fig. 6, the abrasive layer 111 has an average thickness of 100 $\mu$m, and the base 112 is made of nickel and structured like a shell. The abrasive layer 111 is made of diamond abrasive grains, and the rotating abrasive head 11 is shaped like a spindle tapered at both ends. Moreover, the rotating abrasive head 11 defines a minimum diameter at the distal portion 11a, which allows for better atherectomy and drilling into a stenotic portion. The radius of curvature of the distal portion 11a of the rotating abrasive head 11 is 3 mm, and the radius of curvature of the proximal portion 11b of the rotating abrasive head 11 is 3 mm. The minimum diameter of the rotating abrasive head 11 defined at the distal portion 11a is 0.20 mm, and the maximum diameter of the intermediate portion 11c of the rotating abrasive head 11 is 2.0 mm. The minimum diameter of the proximal portion 11b of the rotating abrasive head 11 is 1.0 mm. This embodiment operates in a similar way to that of the embodiment shown in Figs. 4 and 5 and, therefore, needs not be described in further detail herein.

Referring to Figs. 4 and 5, the rotational atherectomy device 10 may be delivered to an in vivo target site through a catheter 41. Additionally, a liquid coolant and/or lubricant (typically saline or other biocompatible liquids) may be supplied to the rotational atherectomy device 10 through a passage in the catheter 41.

In summary, the rotational atherectomy device and apparatus have at least the following advantages:

1) Arranging the rotating abrasive head at the distal end of the drive shaft imparts atherectomy capabilities to the distal end of the rotational atherectomy device. Accordingly, in a surgical procedure, it is made unnecessary to screw or push a distal drive shaft section through a stenotic lesion. Instead, the rotating abrasive head can be directly brought into contact with the lesion for atherectomy. In this way, better atherectomy performance and crossability can be achieved, reducing the risk of the surgical procedure and increasing its success rate.

2) Deviating the centroid of the structure consisting of the connecting portion of the drive shaft and the rotating abrasive head coupled thereto enables the rotating abrasive head to adapt a diameter of atherectomy to a lesion in need of atherectomy and recanalization, thereby allowing for effective removal of the lesion. This dispenses with the use of multiple rotating abrasive heads of various sizes, which requires frequent interchanging of associated devices and a longer surgical time and is associated with a higher surgical risk. In particular, the rotating abrasive head is allowed to have a smaller static diameter, which enables the rotational atherectomy device to be more easily advanced through a narrow blood vessel and catheter to a target vascular lesion, reducing surgical difficulties. Further, a smaller static diameter of the rotating abrasive head is associated with a lower probability of the rotating abrasive head blocking blood flow in a stenotic blood vessel or blocking distal flow of a liquid coolant and/or lubricant. This further reduces the risk associated with a surgical procedure and increases its safety.

3) As only one rotating abrasive head is included and disposed at the distal end of the drive shaft, the drive shaft is allowed to have a reduced axial length, which mitigates the influence of the drive shaft on distal side of a lesion in need of atherectomy and normal blood vessel segments on proximal side. In particular, the single rotating abrasive head is eccentrically disposed to allow for controlled centrifugation during atherectomy, which does not adversely affect movement of the rotating abrasive head or a blood vessel. In this way, uncertainties associated with a surgical procedure using the device can be reduced, increasing safety of the surgical procedure. Moreover, the use of only one rotating abrasive head does not increase overall stiffness of the drive shaft and ensures that it is compliant enough to facilitate its advancement through a blood vessel and catheter.

Further, when the rotating abrasive head is coated on its entire outer surface with the abrasive layer, it is capable of bidirectional atherectomy. Thus, even when the rotating abrasive head gets stuck in a lesion during its forward movement under the action of a pushing force, it can be retracted while abrading the lesion. As a result, not only more efficient atherectomy can be achieved, the risk of a surgical procedure using the device can be further reduced.

It will be understood that, since the centroid of the structure does not coincide with the center axes of the hollow cavities, as the rotating abrasive head and the drive shaft are spun about the center axes of the hollow cavities, the rotating abrasive head will revolve under the action of a centrifugal force about an axis deviated from the center axes of the hollow cavities. Moreover, the diameter of revolution of the rotating abrasive head can be controlled by adjusting its speed of rotation. For example, the diameter of atherectomy may increase with the speed of rotation beyond the static diameter of the rotating abrasive head. In this way, the rotating abrasive head is allowed to have a smaller static diameter, and the use of multiple rotating abrasive heads of various static diameters can be avoided, which may require interchanging of these rotating abrasive heads during surgery. Further, the diameter of atherectomy that is larger than the static diameter of the rotating abrasive head can facilitate flow of blood, a liquid coolant and/or a liquid lubricant to flow around the rotating abrasive head and reduce the risk of the rotating abrasive head blocking blood flow in a blood vessel.

It will be understood that while a few preferred embodiments of the present invention have been described above, the scope of the invention is in no way limited to these disclosed embodiments. Any and all changes made to the configurations of the above embodiments are intended to fall within the scope of the present invention. Those skilled in the art can devise other embodiments on the basis of the description of the foregoing embodiments.

**Claims**

1. A rotational atherectomy device for use in vascular surgery, comprising a drive shaft and a rotating abrasive head, the drive shaft provided with a connecting portion located at a distal end, the connecting portion connected to the rotating abrasive head, the rotating abrasive head and the drive shaft separately defining hollow cavities axially extending therethrough, the hollow cavities configured for insertion of a guide body therethrough, wherein a centroid of a structure consisting of the rotating abrasive head and the connecting portion connected thereto does not coincide with center axes of the hollow cavities.

2. The rotational atherectomy device according to claim 1, wherein a diameter of a distal portion of the rotating abrasive head gradually increases from distal to proximal in an axial direction, and an outer surface of the distal portion of the rotating abrasive head is coated with an abrasive layer.

3. The rotational atherectomy device according to claim 2, wherein a diameter of a proximal portion of the rotating abrasive head gradually decreases from distal to proximal in the axial direction, and an outer surface of the proximal portion of the rotating abrasive head is coated with the abrasive layer, and/or an outer surface of an intermediate portion of the rotating abrasive head between the distal portion and the proximal portion is coated with the abrasive layer.

4. The rotational atherectomy device according to claim 3, wherein the intermediate portion has a constant diameter, or wherein a diameter of the intermediate portion gradually increasing and then gradually decreasing from distal to proximal in the axial direction.

5. The rotational atherectomy device according to any one of claims 2 to 4, wherein the abrasive layer has a thickness of 20-120 μm.

6. The rotational atherectomy device according to any one of claims 1 to 4, wherein a diameter of the rotating abrasive head gradually decreases distally in the axial direction and the diameter of the rotating abrasive head gradually decreases proximally in the axial direction.

7. The rotational atherectomy device according to claim 6, wherein the rotating abrasive head comprises at least one of:

a minimum diameter of the distal portion of the rotating abrasive head smaller than a minimum diameter of the proximal portion of the rotating abrasive head;
a maximum diameter of the distal portion of the rotating abrasive head smaller than a maximum diameter of the proximal portion of the rotating abrasive head; and
a radius of curvature of the distal portion of the rotating abrasive head greater than or equal to a radius of curvature of the proximal portion of the rotating abrasive head.

8. The rotational atherectomy device according to claim 6, wherein the rotating abrasive head comprises at least one of:

a diameter of the distal portion of the rotating abrasive head is 0.13-0.66 mm;
a maximum diameter of the intermediate portion between the proximal portion and the distal portion of the rotating abrasive head is 0.66-4.0 mm; and
a diameter of the proximal portion of the rotating abrasive head is 0.5-1.2 mm.

9. The rotational atherectomy device according to any one of claims 1 to 4, further comprising the guide body, wherein the rotating abrasive head and the drive shaft are rotatable and axially movable relative to the guide body.

10. The rotational atherectomy device according to any one of claims 1 to 4, wherein the hollow cavity of the rotating abrasive head consists of a distal hollow cavity section and a proximal hollow cavity section in communication with each other, the distal hollow cavity section having a diameter matching a diameter of the guide body, the proximal hollow cavity section having a diameter greater than the diameter of the distal hollow cavity section, the proximal hollow cavity section secured to the connecting portion.

11. The rotational atherectomy device according to claim 10, wherein the drive shaft is generally a hollow tubular structure with a constant diameter, and wherein a center axis of the drive shaft is coincident with a center axis of the guide body.

12. The rotational atherectomy device according to claim 10, wherein the drive shaft is a hollow tubular structure with a varying diameter, wherein the connecting portion has a diameter increasing from proximal to distal in the axial direction, and wherein the drive shaft further has a constant-diameter section joined to a proximal end of the connecting portion, the constant-diameter section having a center axis coincident with a center axis of the guide body.

13. The rotational atherectomy device according to claim 12, wherein the connecting portion is inserted into the proximal hollow cavity section through a proximal opening of the rotating abrasive head, the proximal opening having a radial dimension smaller than or equal to a diameter of a proximal end of the connecting portion.

14. The rotational atherectomy device according to claim 13, wherein the connecting portion is configured to be asymmetric about the center axis of the constant-diameter section, and/or wherein the connecting portion has a diameter gradually increasing and then gradually decreasing from proximal to distal in the axial direction.

15. A rotational atherectomy apparatus, comprising a drive device and the rotational atherectomy device of any one of claims 1 to 14, the drive device coupled to the drive shaft in the rotational atherectomy device and configured to actuate rotation of the rotational atherectomy device.

Fig. 1

Fig. 2

Fig. 3

Fig. 4

Fig. 5

Fig. 6

Centrifugal Force Fc

Speed of Rotation n

Fig. 7

Centrifugal Force Fc

Diameter of Atherectomy D

Fig. 8

## INTERNATIONAL SEARCH REPORT

| International application No. |
|---|
| **PCT/CN2023/094969** |

**A. CLASSIFICATION OF SUBJECT MATTER**

A61B17/3207(2006.01)i

According to International Patent Classification (IPC) or to both national classification and IPC

**B. FIELDS SEARCHED**

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; CNKI; ENTXTC; ENTXT; OETXT; VEN: 质心, 偏心, 离心, 不重合, 不对称, 非对称, 磨, 挫, 削, 切, 旋转, 转动, 驱动轴, eccentric, centroid, acentric, dissymmetrical, unsymmetrical, abrade, cut, rotate, turn, drive

**C. DOCUMENTS CONSIDERED TO BE RELEVANT**

| Category* | Citation of document, with indication, where appropriate, of the relevant passages | Relevant to claim No. |
|---|---|---|
| PX | CN 114916997 A (SHANGHAI MINIMAL-INVASIVE MELODY MEDICAL TECHNOLOGY CO., LTD.) 19 August 2022 (2022-08-19) claims 1-15, description, paragraphs [0005]-[0089], and figures 1-8 | 1-15 |
| PX | CN 217645302 U (SHANGHAI MINIMAL-INVASIVE MELODY MEDICAL TECHNOLOGY CO., LTD.) 25 October 2022 (2022-10-25) claims 1-15, description, paragraphs [0005]-[0089], and figures 1-8 | 1-15 |
| X | US 5681336 A (BOSTON SCIENT CORP.) 28 October 1997 (1997-10-28) description, column 3, line 60 to column 4, line 46, and figures 1 and 4 | 1-9, 15 |
| Y | US 5681336 A (BOSTON SCIENT CORP.) 28 October 1997 (1997-10-28) description, column 3, line 60 to column 4, line 46, and figures 1 and 4 | 10-15 |
| Y | US 2015245851 A1 (REX MEDICAL LP) 03 September 2015 (2015-09-03) description, paragraphs [0068]-[0078], and figures 1-4 | 10-15 |
| X | US 2016157886 A1 (BOSTON SCIENT SCIMED INC.) 09 June 2016 (2016-06-09) description, paragraphs [0045]-[0080], and figures 1-5 | 1-15 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 August 2023** | **28 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/094969** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 102300510 A (CARDIOVASCULAR SYSTEMS, INC.) 28 December 2011 (2011-12-28)<br>entire document | 1-15 |
| A | US 5314438 A (SHTURMAN CARDIOLOGY SYSTEMS, INC.) 24 May 1994 (1994-05-24)<br>entire document | 1-15 |
| A | US 6132444 A (SHTURMAN CARDIOLOGY SYSTEMS, INC.) 17 October 2000<br>(2000-10-17)<br>entire document | 1-15 |

Form PCT/ISA/210 (second sheet) (July 2022)

## INTERNATIONAL SEARCH REPORT
### Information on patent family members

International application No.

**PCT/CN2023/094969**

| Patent document cited in search report | | | Publication date (day/month/year) | Patent family member(s) | | | Publication date (day/month/year) |
|---|---|---|---|---|---|---|---|
| CN | 114916997 | A | 19 August 2022 | None | | | |
| CN | 217645302 | U | 25 October 2022 | None | | | |
| US | 5681336 | A | 28 October 1997 | None | | | |
| US | 2015245851 | A1 | 03 September 2015 | US | 10271869 | B2 | 30 April 2019 |
| US | 2016157886 | A1 | 09 June 2016 | US | 10405879 | B2 | 10 September 2019 |
| CN | 102300510 | A | 28 December 2011 | CN | 102300510 | B | 30 July 2014 |
| US | 5314438 | A | 24 May 1994 | None | | | |
| US | 6132444 | A | 17 October 2000 | None | | | |

Form PCT/ISA/210 (patent family annex) (July 2022)